# EUROPEAN PATENT APPLICATION

(11) **EP 3 792 927 A1**
(43) Date of publication of application: **17.03.2021**
(21) Application number: 20196031.7
(22) Date of filing: 14.09.2020
(51) Int. Cl.: G16H 15/00, G16H 10/40, G16H 50/30, G16H 10/60

(54) **METHOD AND APPARATUS FOR PROVIDING REAL-TIME PERIODIC HEALTH UPDATES**

(30) Priority: 13.09.2019 US 201916570558
(71) Applicant: Honeywell International Inc., Charlotte, NC 28202 (US)
(72) Inventor: ARORA, Abhinav, Charlotte, NC North Carolina 28202 (US); MAHESHWARI, Shikha, Charlotte, NC North Carolina 28202 (US)
(74) Representative: Dennemeyer & Associates S.A.

(57) **Abstract**

Various embodiments enable generation of a detailed health report for a patient based at least in part on patient data collected and/or generated at a wearable client device. The patient data may be generated by one or more sensors onboard and/or in communication with the wearable client device. The patient data is provided to a centralized monitoring system, which analyzes the patient data in accordance with user input provided by one or more healthcare providers having access to anonymized versions of the patient data. The monitoring system then generates the detailed health report based at least in part on the results of the analysis performed on the patient data.

## Description

### BACKGROUND

In an effort to control the cost of healthcare, health trackers and remote patient monitoring devices have been used to monitor patient health, such as monitoring basic health characteristics, monitoring symptoms of chronic diseases, monitoring symptoms that could develop into serious conditions, and/or the like. However, ensuring data privacy is fully maintained, and user consent is obtained prior to transmitting any user's health data to enable remote monitoring have posed challenges to implementing wearable health tracking technologies.

Various health monitoring programs exist. However, improvements may yet be desired for health monitoring devices and/or systems that provide effective data collection and identification of a patient's risk for developing health issues, and/or which enable healthcare professionals to provide additional insight into patient data, even if that data is generated remotely. Further, there is a need to provide robust and convenient health data monitoring via devices and/or systems that may collect and transfer data as well as monitor events in real-time, including multi-variable parameter determination. Through applied effort, ingenuity, and innovation, many of these identified problems have been solved by developing solutions that are included in embodiments of the present invention, many examples of which are described in detail herein.

### BRIEF SUMMARY

In general, embodiments of the present invention provide methods, apparatuses, systems, computing devices, computing entities, and/or the like for monitoring a plurality of patient characteristics, such as physiological parameters of the patient (represented by patient data) of one or more individual users/patients collected by one or a plurality of sensors in comparison to metric data (e.g., providing baseline characteristic data and providing real-time periodic health updates for users/patients). Certain embodiments provide for "on-patient" portable and real-time continuous multi-parameter medical assessment and evaluation capabilities so as to alert the patient and/or medical professionals that the patient should seek medical help, if necessary. In another embodiment, machine-learning based models are utilized to train a monitoring apparatus of most-applicable patient data for patients and healthcare professionals to maximize the predicted likelihood that the patient will need appropriate medical treatment.

In accordance with one aspect, a method, computer program product, and apparatus is provided. In one embodiment, the method comprises receiving metric data defining one or more patient characteristics and test data types for monitoring each of the one or more patient characteristics; transmitting the metric data to a client device associated with a patient, wherein the client device is configured to receive test data matching the test data types for monitoring each of the one or more patient characteristics; receiving, from the client device, patient data comprising the test data received at the client device; generating a health report comprising health values corresponding to the one or more patient characteristics based at least in part on the patient data; and transmitting the health report to the client device.

Various embodiments are directed to an apparatus comprising at least one processor and at least one memory including computer program code for one or more programs, the at least one memory and the computer program code configured to, with the at least one processor, cause the apparatus to: receive metric data defining one or more patient characteristics and test data types for monitoring each of the one or more patient characteristics; transmit the metric data to a client device associated with a patient, wherein the client device is configured to receive test data matching the test data types for monitoring each of the one or more patient characteristics; receive, from the client device, patient data comprising the test data received at the client device; generate a health report comprising health values corresponding to the one or more patient characteristics based at least in part on the patient data; and transmit the health report to the client device.

In certain embodiments, the client device is a wearable client device. Moreover, the wearable client device may be configured for generating at least a portion of the test data. In various embodiments, the at least one memory and the computer program code are further configured to, with the at least one processor, cause the apparatus to perform at least the following: update the metric data based at least in part on a state of the patient, a state of the patient's environment, and a monitoring model; and transmit the updated metric data to the client device. In various embodiments, the test data types comprise one or more of blood test results and vitals monitoring. Moreover, in certain embodiments, the at least one memory and the computer program code are further configured to, with the at least one processor, cause the apparatus to perform at least the following: generate a health report presenting at least a portion of the patient data to a healthcare professional; receiving input from the healthcare professional; and determining health values based at least in part on the input from the healthcare professional. Moreover, in certain embodiments, the patient data is identified by a patient identifier identifying the patient at a monitoring system. In various embodiments, the at least one memory and the computer program code are further configured to, with the at least one processor, cause the apparatus to perform at least the following: provide monitoring services to a plurality of users in a plurality of test data processing steps provided by the monitoring system, resulting in a plurality of health report views.

Certain embodiments are directed to a computer-implemented method, the method comprising: receiving metric data defining one or more patient characteristics and test data types for monitoring each of the one or more patient characteristics; transmitting the metric data to a client device associated with a patient, wherein the client device is configured to receive the test data matching the test data types for monitoring each of the one or more patient characteristics; receiving, from the client device, patient data comprising the test data received at the client device; generating a health report comprising health values corresponding to the one or more patient characteristics based at least in part on the patient data; and transmitting the health report to the client device.

In various embodiments, the client device is a wearable client device. In certain embodiments, the wearable client device is configured for generating at least a portion of the test data. Moreover, in certain embodiments, the method further comprises: updating the metric data based at least in part on a state of the patient, a state of the patient's environment, and a monitoring model; and transmitting the updated metric data to the client device. In various embodiments, the test data types comprise one or more of blood test results and vitals monitoring. The method may also comprise: generating a health report comprising presenting at least a portion of the patient data to a healthcare professional; receiving input from the healthcare professional; and determining health values based at least in part on the input. In certain embodiments, the patient data is identified by a patient identifier identifying the patient at a monitoring system. In various embodiments, the method further comprises providing monitoring services to a plurality of users in a plurality of test data processing steps provided by the monitoring system, resulting in a plurality of health report views.

Certain embodiments are directed to a computer program product comprising at least one non-transitory computer-readable medium having computer-readable program instructions stored therein, the computer-readable program instructions comprising instructions, which when performed by an apparatus, are configured to cause the apparatus to at least perform: receive metric data defining one or more patient characteristics and test data types for monitoring each of the one or more patient characteristics; transmit the metric data to a client device associated with a patient, wherein the client device is configured to receive test data matching the test data types for monitoring each of the one or more patient characteristics; receive, from the client device, patient data comprising the test data received at the client device; generate a health report comprising health values corresponding to the one or more patient characteristics based at least in part on the patient data; and transmit the health report to the client device.

In certain embodiments, the client device is a wearable client device. Moreover, in various embodiments, the wearable client device is configured for generating at least a portion of the test data. In certain embodiments, the at least one non-transitory computer-readable medium having computer-readable program instructions stored therein, the computer-readable program instructions comprising further instructions, which when performed by the apparatus, are configured to further cause the apparatus to at least perform: update the metric data based at least in part on a state of the patient, a state of the patient's environment, and a monitoring model; and transmit the updated metric data to the client device.

### BRIEF DESCRIPTION OF THE SEVERAL VIEWS OF THE DRAWING(S)

Having thus described the invention in general terms, reference will now be made to the accompanying drawings, which are not necessarily drawn to scale, and wherein:
Fig. 1 is an exemplary overview of a system that can be used to practice embodiments of the present invention;
Fig. 2 illustrates an example server device in accordance with some embodiments discussed herein;
Fig. 3 illustrates an example client device in accordance with some embodiments discussed herein;
Fig. 4 illustrates a flow diagram of an example system in accordance with some embodiments discussed herein; and
Fig. 5 illustrates data flows enabling operation of various embodiments.

### DETAILED DESCRIPTION

Various embodiments of the present invention now will be described more fully hereinafter with reference to the accompanying drawings, in which some, but not all embodiments of the inventions are shown. Indeed, these inventions may be embodied in many different forms and should not be construed as limited to the embodiments set forth herein; rather, these embodiments are provided so that this disclosure will satisfy applicable legal requirements. The term "or" is used herein in both the alternative and conjunctive sense, unless otherwise indicated. The terms "illustrative" and "exemplary" are used to be examples with no indication of quality level. Like numbers refer to like elements throughout.

### I. Overview

Discussed herein are methods, apparatus, systems, computing devices, computing entities, and/or the like for providing real-time/near-real-time/periodic health updates for patients and generating a health report associated with a patient. As will be recognized, however, the disclosed concepts can be used to remind a user to perform any type of task and are not limited to a particular context.

In an example embodiment, methods described herein are configured to transform or otherwise manipulate a user device, such as a wearable client device, so that it functions as a special purpose computing system to provide health reporting and/or health monitoring processes/functionalities, such as by providing scoring, data analytics, and/or machine-learning to provide health report data, such as forecasting health issues or complications for a user/patient. In an example embodiment, the systems and methods described herein are configured to provide analytics for generating an output to a health report relating to a plurality of health characteristics which may be analyzed by medical care providers so as to provide their own analysis. The results of automatically executed analysis and/or analysis performed by medical care providers may be consolidated into a detailed health report for a user/patient, which may be provided to the patient via a graphical user interface accessible via one or more computing entities (e.g., the wearable client device configured for collecting patient data and/or another computing entity).

In some examples, the systems and methods described herein may collect, ingest, or otherwise access patient data via a wearable client device. As just one example, the patient data may be generated locally at the wearable client device, for example, via one or more sensors (e.g., blood pressure sensors, pulse sensors, and/or the like). In other examples, the patient data may be ingested by the wearable client device, for example, from an external sensor device, which may be physically, electronically, and/or wirelessly connected with the wearable client device (e.g., a blood sugar testing device and/or other blood testing devices). In some examples, the systems and methods are configured to evaluate the patient data based at least in part on defined metric data stored at the wearable client device and/or to provide analysis/forecasting of health data for the patient, for example, in the form of a health report accessible to the patient (e.g., via a personalized graphical user interface dashboard accessible to the patient). Accordingly, the provision of such data, a health report, and personalized graphical user interface, and/or the like are advantageous, in some examples, to medical providers who may not have physical access to the patient and/or the patient's collected data (e.g., blood samples) in order to provide their analysis. Moreover, user/patients of various embodiments receive up-to-date analysis of their health as reflected by patient data generated and/or collected by a wearable client device.

Moreover, to provide users/patients with the results of detailed analyses of their patient data as performed by healthcare professionals, embodiments provide the patient data in an anonymized format to those healthcare professionals such that the healthcare professionals are able to perform a detailed analysis of the data provided, without sacrificing the user/patient's privacy. Accordingly, the systems and methods described herein provide for anonymizing patient data that allows for anonymous research and analysis results to be correlated by a healthcare professional. According to some embodiments, monitoring systems are provided as cloud-based systems, particularly, for providing the patient data to a variety of clients, such as healthcare professionals from medical institutes, agents from insurance companies, patients, medical researchers, patient caregivers, patient family members, and/or the like, while adhering to a patient's privacy preferences. The monitoring system has the capability of processing a patient data to allow multiple healthcare professional to view and analyze either independently or in a collaborated manner to identify and monitor health characteristics (e.g., blood pressure, diabetes, thyroid issues, etc.). Dependent upon the privileges associated with their roles (e.g., doctors, insurance agents, patients, or third party data analysts or researchers), different healthcare professional may be limited to access only a portion of information relating to the patient data or a subset without compromising the privacy of the patient.

### II. Computer Program Products, Methods, and Computing Entities

Embodiments of the present invention may be implemented in various ways, including as computer program products that comprise articles of manufacture. Such computer program products may include one or more software components including, for example, software objects, methods, data structures, or the like. A software component may be coded in any of a variety of programming languages. An illustrative programming language may be a lower-level programming language such as an assembly language associated with a particular hardware architecture and/or operating system platform. A software component comprising assembly language instructions may require conversion into executable machine code by an assembler prior to execution by the hardware architecture and/or platform. Another example programming language may be a higher-level programming language that may be portable across multiple architectures. A software component comprising higher-level programming language instructions may require conversion to an intermediate representation by an interpreter or a compiler prior to execution.

Other examples of programming languages include, but are not limited to, a macro language, a shell or command language, a job control language, a script language, a database query or search language, and/or a report writing language. In one or more example embodiments, a software component comprising instructions in one of the foregoing examples of programming languages may be executed directly by an operating system or other software component without having to be first transformed into another form. A software component may be stored as a file or other data storage construct. Software components of a similar type or functionally related may be stored together such as, for example, in a particular directory, folder, or library. Software components may be static (e.g., pre-established or fixed) or dynamic (e.g., created or modified at the time of execution).

A computer program product may include a non-transitory computer-readable storage medium storing applications, programs, program modules, scripts, source code, program code, object code, byte code, compiled code, interpreted code, machine code, executable instructions, and/or the like (also referred to herein as executable instructions, instructions for execution, computer program products, program code, and/or similar terms used herein interchangeably). Such non-transitory computer-readable storage media include all computer-readable media (including volatile and non-volatile media).

In one embodiment, a non-volatile computer-readable storage medium may include a floppy disk, flexible disk, hard disk, solid-state storage (SSS) (e.g., a solid state drive (SSD), solid state card (SSC), solid state module (SSM), enterprise flash drive, magnetic tape, or any other non-transitory magnetic medium, and/or the like. A non-volatile computer-readable storage medium may also include a punch card, paper tape, optical mark sheet (or any other physical medium with patterns of holes or other optically recognizable indicia), compact disc read only memory (CD-ROM), compact disc-rewritable (CD-RW), digital versatile disc (DVD), Blu-ray disc (BD), any other non-transitory optical medium, and/or the like. Such a non-volatile computer-readable storage medium may also include read-only memory (ROM), programmable read-only memory (PROM), erasable programmable read-only memory (EPROM), electrically erasable programmable read-only memory (EEPROM), flash memory (e.g., Serial, NAND, NOR, and/or the like), multimedia memory cards (MMC), secure digital (SD) memory cards, SmartMedia cards, CompactFlash (CF) cards, Memory Sticks, and/or the like. Further, a non-volatile computer-readable storage medium may also include conductive-bridging random access memory (CBRAM), phase-change random access memory (PRAM), ferroelectric random-access memory (FeRAM), non-volatile random-access memory (NVRAM), magnetoresistive random-access memory (MRAM), resistive random-access memory (RRAM), Silicon-Oxide-Nitride-Oxide-Silicon memory (SONOS), floating junction gate random access memory (FJG RAM), Millipede memory, racetrack memory, and/or the like.

In one embodiment, a volatile computer-readable storage medium may include random access memory (RAM), dynamic random access memory (DRAM), static random access memory (SRAM), fast page mode dynamic random access memory (FPM DRAM), extended data-out dynamic random access memory (EDO DRAM), synchronous dynamic random access memory (SDRAM), double data rate synchronous dynamic random access memory (DDR SDRAM), double data rate type two synchronous dynamic random access memory (DDR2 SDRAM), double data rate type three synchronous dynamic random access memory (DDR3 SDRAM), Rambus dynamic random access memory (RDRAM), Twin Transistor RAM (TTRAM), Thyristor RAM (T-RAM), Zero-capacitor (Z-RAM), Rambus in-line memory module (RIMM), dual in-line memory module (DIMM), single in-line memory module (SIMM), video random access memory (VRAM), cache memory (including various levels), flash memory, register memory, and/or the like. It will be appreciated that where embodiments are described to use a computer-readable storage medium, other types of computer-readable storage media may be substituted for or used in addition to the computer-readable storage media described above.

As should be appreciated, various embodiments of the present invention may also be implemented as methods, apparatus, systems, computing devices, computing entities, and/or the like. As such, embodiments of the present invention may take the form of an apparatus, system, computing device, computing entity, and/or the like executing instructions stored on a computer-readable storage medium to perform certain steps or operations. Thus, embodiments of the present invention may also take the form of an entirely hardware embodiment, an entirely computer program product embodiment, and/or an embodiment that comprises combination of computer program products and hardware performing certain steps or operations.

Embodiments of the present invention are described below with reference to block diagrams and flowchart illustrations. Thus, it should be understood that each block of the block diagrams and flowchart illustrations may be implemented in the form of a computer program product, an entirely hardware embodiment, a combination of hardware and computer program products, and/or apparatus, systems, computing devices, computing entities, and/or the like carrying out instructions, operations, steps, and similar words used interchangeably (e.g., the executable instructions, instructions for execution, program code, and/or the like) on a computer-readable storage medium for execution. For example, retrieval, loading, and execution of code may be performed sequentially such that one instruction is retrieved, loaded, and executed at a time. In some exemplary embodiments, retrieval, loading, and/or execution may be performed in parallel such that multiple instructions are retrieved, loaded, and/or executed together. Thus, such embodiments can produce specifically-configured machines performing the steps or operations specified in the block diagrams and flowchart illustrations. Accordingly, the block diagrams and flowchart illustrations support various combinations of embodiments for performing the specified instructions, operations, or steps.

### III. Exemplary System Architecture

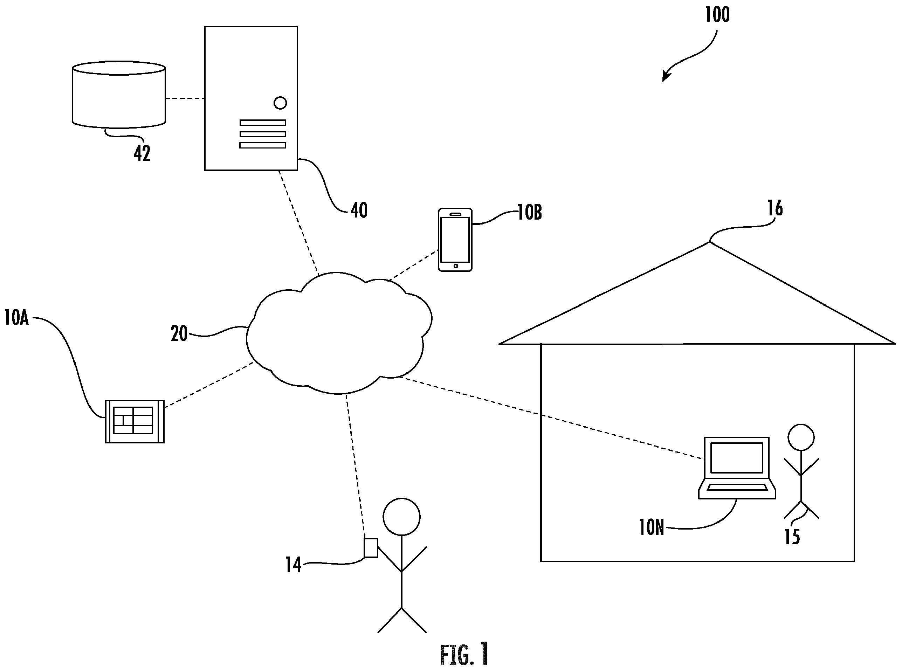

Fig. 1 provides an illustration of an exemplary embodiment of the present invention. Fig. 1 shows system 100 including an example network architecture for a system, which may include one or more devices and sub-systems that are configured to implement embodiments discussed herein. For example, the system 100 may include monitoring system 40, which can include, for example, processing resources, a data storage area (e.g., a database), and/or the like. The monitoring system 40 may include any suitable type of processing device. In some embodiments, the monitoring system 40 may determine and transmit commands and instructions for monitoring models, generating health reports using data stored via a database 42 which may be stored as a part of and/or in communication with one or more client devices 10A-10N and/or the monitoring system 40. The database 42 includes information captured and stored by the client wearable client device 14 to facilitate the operations of the system.

System 100 includes the one or more client devices 10A-10N and client wearable client device 14 configured for communication with the monitoring system 40 via network 20. Client devices 10A-10N may be operated by and/or may represent a variety of entities, including users/patients organizations such as medical institutes, medical care professionals, other individuals, other organizations, and/or the like.

In one embodiment, monitoring system 40 may represent a set of servers or clusters of servers associated with a service provider and geographically distributed over a network. For example, monitoring system 40 may be associated with a medical data processing service provider. Network 20 may be a local area network (LAN), a metropolitan area network (MAN), a wide area network (WAN) such as the Internet or an intranet, or a combination thereof. Monitoring system 40 can be made of a variety of servers and devices capable of providing application services to a variety of clients such as client devices 10A-10N over network 20. In one embodiment, monitoring system 40 includes one or more monitoring systems 40 to provide data processing services, one or more databases 42 to store patient data, health reports, and other medical data, and one or more routers to transfer data to/from other entities such as entities client devices 10A-10N and client wearable client device 14.

Client wearable client device 14 is configured to collect patient data from sensors (e.g., vital signs measurement sensors, accelerometer, oximeter, pressure sensor, gyroscope, temperature sensor, proximity sensor, etc.) that may be formed as a subcomponent of the client wearable client device 14 and/or external sensors that may be in wireless or wired communication with the client wearable client device 14. In certain embodiments, external sensors may be physically connected with the client wearable client device 14 (e.g., detachably connected or permanently connected). In other embodiments, the external sensors may not themselves be wearable, such as laboratory-based blood testing devices. Accordingly, the client wearable client device 14 may comprise one or more test data receivers (wired or wireless) for receiving additional test data from external testers/sensors (e.g., external blood testers, onboard blood testers, etc.).

The client wearable client device 14 is configured to consolidate and/or generate patient data based at least in part on data collected from the various sensors, testers, and/or other data sources. As discussed herein, the client wearable client device 14 may be configured to, as a part of consolidating the patient data, generate and/or collect particular patient data types, consistent with metric data defining patient data types necessary to perform one or more analysis requested for the user/patient. In certain embodiments, the client wearable client device 14 may additionally be configured to automatically analyze at least a portion of the patient data, and to generate at least a portion of a health report to be made accessible to the patient. In certain embodiments, at least a portion of the health report may be available locally at the client wearable client device 14. In other embodiments, the health report may be generated at least in part at the monitoring system 40, and may be provided to the user/patient via one or more client devices 10 and/or the client wearable client device. The patient data collected by the wearable client device along with any analysis outputs generated are transmitted to monitoring system 40 for additional analysis (e.g., via automated analysis at the monitoring system 40, for example, by machine-learning based algorithms, and/or based on manual review by healthcare professionals accessing at least a portion of the patient data via one or more client devices 10A-10N).

With reference again to Fig. 1, database 42 may be a data store to store patient data and/or generated outputs (e.g., health report, personalized risk panel, etc.). In certain embodiments, database 42 may also incorporate encryption capabilities. Database 42 may include multiple databases and/or may be maintained by a third party vendor such as storage providers.

In some embodiments, the monitoring system 40 is configured to provide patient data processing services to client devices 10A-10N operated by healthcare professionals 15 (e.g., physicians) associated with organizations 16 (e.g., medical institutes), agents from insurance companies, patients, medical researchers, and/or the like. The monitoring system 40 of certain embodiments, also referred to as a patient data monitoring and processing server, is configured to host anonymized patient data (e.g., patient data having identifying data removed therefrom, while maintaining data collected from one or more sensors (e.g., and collected at the wearable client device 14) thereby enabling healthcare professionals to review the data and to identify potential health-related issues evident in the data without seeing identifying data about the patient) associated with a plurality of patients to allow one or more individuals, such as users of client devices 10A-10N, to participate in a discussion/processing forum of the anonymized data in a collaborated manner and/or conferencing environment. In another example embodiment, medical care professionals 15 from organizations 16 may view patient data (in an anonymized fashion), manage patient diagnosis, manage treatment information, generate alerts, generate communications, control the client wearable client device 14 or other connected devices. In yet another example embodiment, the monitoring system 40 is configured to manage data access privileges associated with roles (e.g., doctors, insurance agents, patients, or third party data analysts or researchers) such that different participants may be limited to access only a portion of the patient data, thereby maintaining an appropriate level of anonymization within the data or a subset of the anonymized data without compromising the privacy of the patients associated with the anonymized data.

Monitoring system 40 is configured to communicate with one or more client devices 10A-10N, wearable client devices 14, and/or other computing entities via communications network 20, and a plurality of client devices 10A-10N may communicate with one another, with wearable client devices 14, and/or with other computing entities via the network 20. In this regard, communications network 20 may include any wired or wireless communication network including, for example, a wired or wireless local area network (LAN), personal area network (PAN), metropolitan area network (MAN), wide area network (WAN), or the like, as well as any hardware, software and/or firmware required to implement it (such as, e.g., network routers, etc.). For example, communications network 20 may include a cellular telephone, an 802.11, 802.16, 802.20, and/or WiMax network. Further, the communications network 20 may include a public network, such as the Internet, a private network, such as an intranet, or combinations thereof, and may utilize a variety of networking protocols now available or later developed including, but not limited to TCP/IP based networking protocols.

Client devices 10A-10N, client wearable client device 14, and/or monitoring system 40 may be implemented as and/or may comprise one or more computers, computing entities, desktop computers, mobile phones, tablets, phablets, notebooks, laptops, distributed systems, cameras, watches, glasses, ear pieces, wristbands, wearable items/devices, items/devices, input terminals, servers or server networks, blades, gateways, switches, processing devices, processing entities, set-top boxes, relays, routers, network access points, base stations, the like, and/or any combination of devices or entities adapted to perform the functions, operations, and/or processes described herein. The depiction in Fig. 1 of "N" client devices is merely for illustration purposes. Any number of users and/or client devices 10 may be included in the system for accessing and/or implementing aspects of the monitoring system 40 and health monitoring system discussed herein (e.g., via one or more interfaces). In one embodiment, the client devices 10A-10N and/or client wearable client device 14 may be configured to display or provide a health update interface on a display of the client device for viewing, creating, editing, and/or otherwise interacting with one or more health reports, which may be provided or pushed by the monitoring system 40 (and may be stored locally at one or more client devices 10A-10N and/or client wearable client device 14). According to some embodiments, the monitoring system 40 may be configured to cause display or presentation of an interface for viewing, creating, editing, and/or otherwise interacting with one or more patient health updates.

As indicated above, the client devices 10A-10N may be any computing device as defined above. Electronic data received by the monitoring system 40 from the client devices 10A-10N and/or client wearable client device 14 may be provided in various forms and via various methods. For example, the client devices 10A-10N may include desktop computers, laptop computers, smartphones, netbooks, tablet computers, wearables, and the like. In embodiments where a client device 10A-10N is a mobile device, such as a smart phone or tablet, the client device 10A-10N may execute an "app" such as the medical monitoring application to interact with the monitoring system 40. Such apps are typically designed to execute on mobile devices, such as tablets or smartphones. For example, an app may be provided that executes on mobile device operating systems such as iOS®, Android®, or Windows®. These platforms typically provide frameworks that allow apps to communicate with one another and with particular hardware and software components of mobile devices. For example, the mobile operating systems named above each provide frameworks for interacting with location services circuitry, wired and wireless network interfaces, user contacts, and other applications. Communication with hardware and software modules executing outside of the app is typically provided via application programming interfaces (APIs) provided by the mobile device operating system.

Additionally or alternatively, the client device 10A-10N and/or client wearable client device 14 may interact with the monitoring system 40 via a web browser. As yet another example, the client device 10A-10N and/or client wearable client device 14 may include various hardware or firmware designed to interface with the monitoring system 40.

Additionally, a plurality of sensors (which in certain embodiments may be a part of and/or in communication with one or more client devices 10A-10N and/or client wearable client device 14) provide information to the server 40 (e.g., via network 20). For example, the sensors may comprise vital signs measurement sensors, pulse sensors, body temperature sensors, blood pressure sensors, an accelerometer, an oximeter, pressure sensor, gyroscope, temperature sensor, proximity sensor, medication container sensors, weight sensors, motion sensors, blood monitoring sensors, other fluid sensors, and/or the like. It should be understood that these sensors, as well as other sensors discussed herein, are provided merely as examples, and any sensors that may be indicative of the health status, location and/or activity of a patient may be provided.

### A. Exemplary Monitoring system

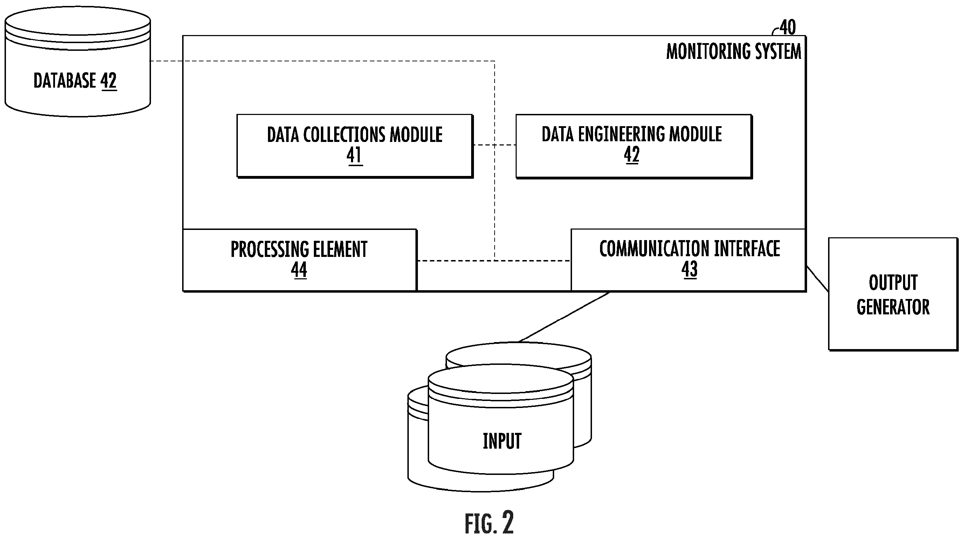

Fig. 2 provides a schematic of a monitoring system 40 according to one embodiment of the present invention. In general, the terms computing entity, entity, device, system, and/or similar words used herein interchangeably may refer to, for example, one or more computers, computing entities, desktop computers, mobile phones, tablets, phablets, notebooks, laptops, distributed systems, items/devices, terminals, servers or server networks, blades, gateways, switches, processing devices, processing entities, set-top boxes, relays, routers, network access points, base stations, the like, and/or any combination of devices or entities adapted to perform the functions, operations, and/or processes described herein. Such functions, operations, and/or processes may include, for example, transmitting, receiving, operating on, processing, displaying, storing, determining, creating/generating, monitoring, evaluating, comparing, and/or similar terms used herein interchangeably. In one embodiment, these functions, operations, and/or processes can be performed on data, content, information, and/or similar terms used herein interchangeably.

Although illustrated as a single computing entity, it should be understood that the monitoring system 40 may be embodied as a plurality of computing entities, tools, and/or the like operating collectively to perform one or more processes, methods, and/or steps. As just one non-limiting example, the monitoring system 40 may comprise a plurality of individual data tools, such as a data collections module 41, a data engineering module 42, and/or the like. Each of these tools may perform specified tasks and/or processes, such that collectively, the monitoring system 40 may be configured to execute one or more tasks requested by a user.

As indicated, in one embodiment, the monitoring system 40 may also include one or more network and/or communications interfaces 43 for communicating with various computing entities, such as by communicating data, content, information, and/or similar terms used herein interchangeably that can be transmitted, received, operated on, processed, displayed, stored, and/or the like. For instance, the monitoring system 40 may communicate with other computing entities, one or more client devices 10A-10N, one or more wearable client devices 14, and/or the like. In certain embodiments, the monitoring system 40 may be configured to receive data from one or more data sources, such as receiving patient data from one or more wearable client devices 14 and/or receiving data indicative of analysis performed by a healthcare professional 15 via a client device 10A-10N, and the monitoring system 40 may be configured to receive data indicative of user input, for example, from a client device 10A-10N.

As shown in Fig. 2, in one embodiment, the monitoring system 40 may include or be in communication with one or more processing elements 44 (also referred to as processors, processing circuitry, and/or similar terms used herein interchangeably) that communicate with other elements within the monitoring system 40 via a bus, for example, or network connection. As will be understood, the processing element 44 may be embodied in a number of different ways. For example, the processing element 44 may be embodied as one or more complex programmable logic devices (CPLDs), microprocessors, multi-core processors, coprocessing entities, application-specific instruction-set processors (ASIPs), and/or controllers. Further, the processing element 44 may be embodied as one or more other processing devices or circuitry. The term circuitry may refer to an entirely hardware embodiment or a combination of hardware and computer program products. Thus, the processing element 44 may be embodied as integrated circuits, application specific integrated circuits (ASICs), field programmable gate arrays (FPGAs), programmable logic arrays (PLAs), hardware accelerators, other circuitry, and/or the like. As will therefore be understood, the processing element 44 may be configured for a particular use or configured to execute instructions stored in volatile or non-volatile media or otherwise accessible to the processing element 44. As such, whether configured by hardware or computer program products, or by a combination thereof, the processing element 44 may be capable of performing steps or operations according to embodiments of the present invention when configured accordingly.

In one embodiment, the monitoring system 40 may further include or be in communication with non-volatile media (also referred to as non-volatile storage, memory, memory storage, memory circuitry and/or similar terms used herein interchangeably). In one embodiment, the non-volatile storage or memory may include one or more non-volatile storage or memory media, which may be implemented as a database 42 and/or may be embodied as hard disks, ROM, PROM, EPROM, EEPROM, flash memory, MMCs, SD memory cards, Memory Sticks, CBRAM, PRAM, FeRAM, RRAM, SONOS, racetrack memory, and/or the like. As will be recognized, the non-volatile storage or memory media may store databases, database instances, database management system entities, data, applications, programs, program modules, scripts, source code, object code, byte code, compiled code, interpreted code, machine code, executable instructions, and/or the like. The term database, database instance, database management system entity, and/or similar terms used herein interchangeably and in a general sense to refer to a structured or unstructured collection of information/data that is stored in a computer-readable storage medium.

Memory media may also be embodied as a data storage device or devices, as a separate database server or servers, or as a combination of data storage devices and separate database servers. Further, in some embodiments, memory media may be embodied as a distributed repository such that some of the stored information/data is stored centrally in a location within the system and other information/data is stored in one or more remote locations. Alternatively, in some embodiments, the distributed repository may be distributed over a plurality of remote storage locations only. An example of the embodiments contemplated herein would include a cloud-based data storage system maintained by a third party provider and where some or all of the information/data.

In one embodiment, the monitoring system 40 may further include or be in communication with volatile media (also referred to as volatile storage, memory, memory storage, memory circuitry and/or similar terms used herein interchangeably). In one embodiment, the volatile storage or memory may also include one or more volatile storage or memory media as described above, such as RAM, DRAM, SRAM, FPM DRAM, EDO DRAM, SDRAM, DDR SDRAM, DDR2 SDRAM, DDR3 SDRAM, RDRAM, RIMM, DIMM, SIMM, VRAM, cache memory, register memory, and/or the like. As will be recognized, the volatile storage or memory media may be used to store at least portions of the databases, database instances, database management system entities, data, applications, programs, program modules, scripts, source code, object code, byte code, compiled code, interpreted code, machine code, executable instructions, and/or the like being executed by, for example, the processing element 44. Thus, the databases, database instances, database management system entities, data, applications, programs, program modules, scripts, source code, object code, byte code, compiled code, interpreted code, machine code, executable instructions, and/or the like may be used to control certain aspects of the operation of the monitoring system 40 with the assistance of the processing element 44 and operating system.

As indicated, in one embodiment, the monitoring system 40 may also include one or more network and/or communications interfaces 208 for communicating with various computing entities, such as by communicating data, content, information, and/or similar terms used herein interchangeably that can be transmitted, received, operated on, processed, displayed, stored, and/or the like. Such communication may be executed using a wired data transmission protocol, such as fiber distributed data interface (FDDI), digital subscriber line (DSL), Ethernet, asynchronous transfer mode (ATM), frame relay, data over cable service interface specification (DOCSIS), or any other wired transmission protocol. Similarly, the monitoring system 40 may be configured to communicate via wireless external communication networks using any of a variety of protocols, such as general packet radio service (GPRS), Universal Mobile Telecommunications System (UMTS), Code Division Multiple Access 2000 (CDMA2000), CDMA2000 IX (1xRTT), Wideband Code Division Multiple Access (WCDMA), Global System for Mobile Communications (GSM), Enhanced Data rates for GSM Evolution (EDGE), Time Division-Synchronous Code Division Multiple Access (TD-SCDMA), Long Term Evolution (LTE), Evolved Universal Terrestrial Radio Access Network (E-UTRAN), Evolution-Data Optimized (EVDO), High Speed Packet Access (HSPA), HighSpeed Downlink Packet Access (HSDPA), IEEE 802.11 (Wi-Fi), Wi-Fi Direct, 802.16 (WiMAX), ultra-wideband (UWB), infrared (IR) protocols, near field communication (NFC) protocols, Wibree, Bluetooth protocols, wireless universal serial bus (USB) protocols, and/or any other wireless protocol. The monitoring system 40 may use such protocols and standards to communicate using Border Gateway Protocol (BGP), Dynamic Host Configuration Protocol (DHCP), Domain Name System (DNS), File Transfer Protocol (FTP), Hypertext Transfer Protocol (HTTP), HTTP over TLS/SSL/Secure, Internet Message Access Protocol (IMAP), Network Time Protocol (NTP), Simple Mail Transfer Protocol (SMTP), Telnet, Transport Layer Security (TLS), Secure Sockets Layer (SSL), Internet Protocol (IP), Transmission Control Protocol (TCP), User Datagram Protocol (UDP), Datagram Congestion Control Protocol (DCCP), Stream Control Transmission Protocol (SCTP), HyperText Markup Language (HTML), and/or the like.

As will be appreciated, one or more of the monitoring system's 40 components may be located remotely from other monitoring system 40 components, such as in a distributed system. Furthermore, one or more of the components may be aggregated and additional components performing functions described herein may be included in the monitoring system 40. Thus, the monitoring system 40 can be adapted to accommodate a variety of needs and circumstances.

### B. Exemplary Client Device

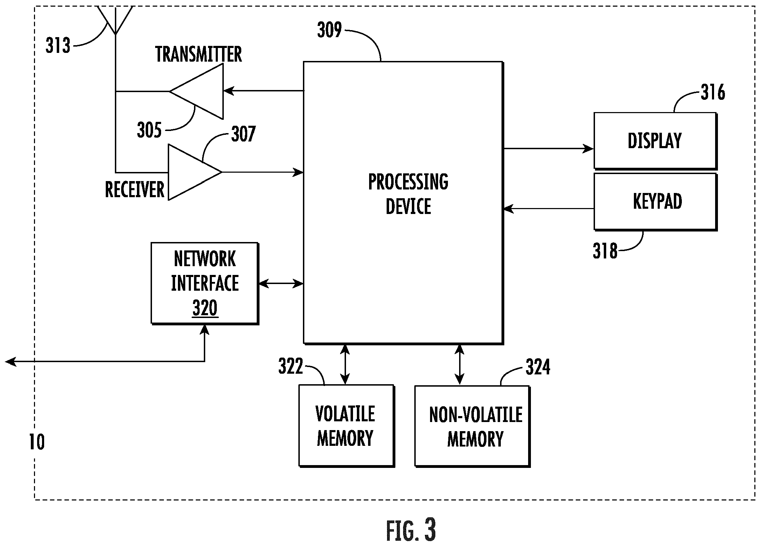

Fig. 3 provides an illustrative schematic representative of client device 10A-10N and client wearable client device 14 that can be used in conjunction with embodiments of the present invention. As shown in Fig. 3, a client device 10 can include an antenna 313, a transmitter 305 (e.g., radio), a receiver 307 (e.g., radio), and a processing element 309 that provides signals to and receives signals from the transmitter 305 and receiver 307, respectively. The signals provided to and received from the transmitter 305 and the receiver 307, respectively, may include signaling information/data in accordance with an air interface standard of applicable wireless systems to communicate with various entities, such as a monitoring system 40, another client device 10, and/or the like. In this regard, the client device 10 may be capable of operating with one or more air interface standards, communication protocols, modulation types, and access types. More particularly, the client device 10 may operate in accordance with any of a number of wireless communication standards and protocols. In a particular embodiment, the client device 10 may operate in accordance with multiple wireless communication standards and protocols, such as GPRS, UMTS, CDMA2000, 1xRTT, WCDMA, TD-SCDMA, LTE, E-UTRAN, EVDO, HSPA, HSDPA, Wi-Fi, WiMAX, UWB, IR protocols, Bluetooth protocols, USB protocols, and/or any other wireless protocol.

Via these communication standards and protocols, the client device 10 can communicate with various other entities using concepts such as Unstructured Supplementary Service information/data (USSD), Short Message Service (SMS), Multimedia Messaging Service (MMS), Dual-Tone Multi-Frequency Signaling (DTMF), and/or Subscriber Identity Module Dialer (SIM dialer). The client device 10 can also download changes, add-ons, and updates, for instance, to its firmware, software (e.g., including executable instructions, applications, program modules), and operating system.

According to one embodiment, the client device 10 may include location determining aspects, devices, modules, functionalities, and/or similar words used herein interchangeably. For example, the client device 10 may include outdoor positioning aspects, such as a location module adapted to acquire, for example, latitude, longitude, altitude, geocode, course, direction, heading, speed, UTC, date, and/or various other information/data. In one embodiment, the location module can acquire data, sometimes known as ephemeris data, by identifying the number of satellites in view and the relative positions of those satellites. The satellites may be a variety of different satellites, including LEO satellite systems, DOD satellite systems, the European Union Galileo positioning systems, the Chinese Compass navigation systems, Indian Regional Navigational satellite systems, and/or the like. Alternatively, the location information/data may be determined by triangulating the computing entity's position in connection with a variety of other systems, including cellular towers, Wi-Fi access points, and/or the like. Similarly, the client device 10 may include indoor positioning aspects, such as a location module adapted to acquire, for example, latitude, longitude, altitude, geocode, course, direction, heading, speed, time, date, and/or various other information/data. Some of the indoor aspects may use various position or location technologies including RFID tags, indoor beacons or transmitters, Wi-Fi access points, cellular towers, nearby computing devices (e.g., smartphones, laptops) and/or the like. For instance, such technologies may include iBeacons, Gimbal proximity beacons, BLE transmitters, NFC transmitters, and/or the like. These indoor positioning aspects can be used in a variety of settings to determine the location of someone or something to within inches or centimeters.

The client device 10 may also comprise a user interface device comprising one or more user input/output interfaces (e.g., a display 316 and/or speaker/speaker driver coupled to a processing element 309 and a touch screen, keyboard, mouse, and/or microphone coupled to a processing element 309). For example, the user output interface may be configured to provide an application, browser, user interface, dashboard, webpage, and/or similar words used herein interchangeably executing on and/or accessible via the client device 10 to cause display or audible presentation of information/data and for user interaction therewith via one or more user input interfaces. As just one specific example, the client device 10 may be configured to output various interface screens associated with a heath monitoring application, which may provide various setup/registration screens and/or may provide one or more reminder prompts or health status alerts for a user of the client device. The user input interface can comprise any of a number of devices allowing the client device 10 to receive data, such as a keypad 318 (hard or soft), a touch display, voice/speech or motion interfaces, scanners, readers, or other input device. In embodiments including a keypad 318, the keypad 318 can include (or cause display of) the conventional numeric (0-9) and related keys (#, *), and other keys used for operating the client device 10 and may include a full set of alphabetic keys or set of keys that may be activated to provide a full set of alphanumeric keys. In addition to providing input, the user input interface can be used, for example, to activate or deactivate certain functions, such as screen savers and/or sleep modes. Through such inputs the client device 10 can collect information/data, user interaction/input, and/or the like.

The client device 10 can also include volatile storage or memory 322 and/or non-volatile storage or memory 324, which can be embedded and/or may be removable. For example, the non-volatile memory may be ROM, PROM, EPROM, EEPROM, flash memory, MMCs, SD memory cards, Memory Sticks, CBRAM, PRAM, FeRAM, RRAM, SONOS, racetrack memory, and/or the like. The volatile memory may be RAM, DRAM, SRAM, FPM DRAM, EDO DRAM, SDRAM, DDR SDRAM, DDR2 SDRAM, DDR3 SDRAM, RDRAM, RIMM, DIMM, SIMM, VRAM, cache memory, register memory, and/or the like. The volatile and non-volatile storage or memory can store databases, database instances, database management system entities, data, applications, programs, program modules, scripts, source code, object code, byte code, compiled code, interpreted code, machine code, executable instructions, and/or the like to implement the functions of the client device 10. Again, as a specific example, the client device memory storage areas (encompassing one or both of the volatile memory 322 and/or non-volatile memory 324) may store the health monitoring application thereon, which itself may encompass a health model/strategy trained for providing optimal health prompts to the user via one or more machine-learning algorithms. As discussed herein, the health model/strategy to be implemented with a particular user is indicative of when particular health characteristics (e.g., blood pressure, blood sugar, heart rate) are above a desirable threshold prompting the user to seek medical attention. The health model/strategy may change over time based on the results of the current health status.

In additional to the components discussed above, a wearable client device 14 may comprise one or more sensors, such as accelerometers, pulse sensors, blood pressure sensors, and/or the like for monitoring the health vitals of a user. As discussed herein, the wearable client device 14 is configured to utilize the output of these onboard sensors to generate at least a portion of patient data, which may comprise raw data output from the onboard sensors, and/or may comprise summary data, as reflected within metric data stored on the wearable client device 14. Moreover, the wearable client device 14 may be configured to receive additional data from one or more testers, sensors, and/or the like that are external to the wearable client device 14. In certain embodiments, the wearable client device 14 may comprise one or more physical connections configured to engage connections of corresponding sensors so as to enable both physical and electronic connection of various sensors. In various embodiments, such sensors may comprise blood monitoring sensors such as glucose sensors, hemoglobin sensors, hematology sensors, and/or the like that have onboard configurations for monitoring blood provided thereto (e.g., detecting blood-related values that may be utilized to determine, for example, blood glucose levels, hemoglobin levels, liver function test results, kidney function test results, and/or other characteristics of a patient's blood that may be indicative of one or more blood-borne diseases, such as Hepatitis, Dengue, Typhoid, Malaria, and/or the like. Data from these external sensors may then be passed through applicable data communication channels (e.g., hardwired through the physical connections) to the wearable client device 14 for generation of corresponding patient data. As yet other examples, the wearable client device 14 may comprise one or more additional communication channels/interfaces as discussed above for receiving other data generated by other external sensors, testers, and/or the like that may not be physically connected to the wearable client device 14. For example, data generated by one or more laboratory equipment may be provided to the wearable client device 14 (e.g., directly and/or indirectly, via one or more user computing entities 10A-10N in communication with the wearable client device 14) via a network connection. In such embodiments, the wearable client device 14 may be configured to generate patient data in accordance with onboard metric data based at least in part on the data received from the external sensors.

### IV. Exemplary System Operation

The operation of various embodiments of the present invention will now be described. As discussed herein, various embodiments are directed to systems and methods for providing users with detailed health data provided within health reports based at least in part on patient data generated and/or provided via a wearable client device 14 of the patient. The detailed health data report may comprise data generated automatically, for example, via automated data analysis performed by a computing entity, such as the wearable client device 14, the monitoring system 40 (e.g., via machine-learning based analysis and/or other automated analysis) and/or data generated based at least in part on user input provided by a healthcare professional, for example, reviewing at least a portion of the patient data (e.g., an anonymized portion of the patient data).

Health reports may be generated for users/patients based on a variety of patient data types. In certain embodiments, health reports may be generated based at least in part on health vitals data generated for the patient, such as generated based at least in part on the patient's blood pressure, pulse, weight, level of activity, and/or the like. Health reports according to certain embodiments may also be generated based at least in part on other patient data, such as patient data generated and/or collected by one or more sensors, for example, sensors analyzing patient bodily fluids (e.g., blood), X-Rays, imaging devices, and/or the like.

In certain embodiments, the content of a health report may be specified based at least in part on user input provided by the user/patient, a healthcare professional, and/or the like. Based at least in part on the identified content of the health report to be provided for a user/patient, the monitoring system 40 may be configured to generate metric data specifying the type of patient data (e.g., raw patient data, also referred to as test data) necessary to enable appropriate analysis to generate the specified content of the health report. In certain embodiments, the metric data defines one or more patient characteristics for which a health report is to be generated, such as identifying a patient's glucose level, a patient's blood pressure, a patient's pulse, a patient's eyesight, and/or the like. Moreover, the metric data identifies test data types (e.g., types of raw patient data generated by one or more sensors) necessary to provide data indicative of the identified patient characteristics. For example, if a health report is to include data indicative of a patient's glucose levels, the metric data generated at the monitoring system 40 may indicate that patient data identifying blood glucose readings is necessary for generation of the health report data, which may comprise health values such as numerical values of blood glucose levels. In certain embodiments, metric data necessary for generation of particular health report data may be identified automatically, for example, based at least in part on machine-learning based methodologies analyzing previously generated health reports and the patient data associated therewith. In other embodiments, metric data may be generated based at least in part on manual user input specifying the patient data necessary to generate particular health report data.

### A. User Registration

In certain embodiments, utilization of a full suite of functionalities of the monitoring system 40 may be limited to user/patients registered therewith. The registration process may provide the monitoring system 40 with basic data necessary for the functionality of various embodiments, such as an identification of a wearable client device 14 identified as providing patient data associated with the user/patient, an identification of contents of a health report requested by the user/patient, and/or the like. Moreover, the registration process may additionally comprise steps for receiving user input indicative of identifying data of the user/patient, that may be stored at the monitoring server 40 for various uses. As just one example, the identifying data may comprise a user/patient name (or other user/patient identifying data, such as a user name or other unique identifier), contact information (e.g., email address, phone number, social media handle, and/or the like). Moreover, the identifying data provided during the registration process may additionally comprise data indicative of user preferences regarding privacy of the patient data (e.g., identifying whether patient data can be provided on an anonymized or identifiable basis to one or more healthcare professionals 15 to aid in analysis of the patient data). The user preferences regarding privacy of the patient data may be provided in a highly granular fashion in certain embodiments, as the user may specify one or more specific healthcare professionals 15 that may see identifying data about the patient in addition to the patient data; the user may specify one or more healthcare professional types (e.g., physician, insurance agent, and/or the like) that may see identifying data about the patient in addition to the patient data; and/or the like. Similarly, the user/patient may specify types of healthcare professionals who can see any patient data about the patient at all.

In certain embodiments, the monitoring system 40 may be configured to identify one or more report data types that may be available for generation for the user/patient based at least in part on the user/patient-specified privacy preferences. Certain data analysis necessary to provide certain report data types may require manual review by a particular healthcare professional type, and accordingly if a user/patient indicates within privacy settings that the user/patient does not desire that particular healthcare professional type to have access to the patient data, then the monitoring system 40 may be configured to inform the user/patient (via user interfaces displayed during the registration process) that certain data analysis is unavailable to the user/patient.

In certain embodiments, the user registration process may be completed at least partially via one or more graphical user interfaces provided via a client device 10A-10N presented to the user/patient and/or via one or more graphical user interfaces provided via the wearable client device 14. The graphical user interfaces provided to the user may be configured to receive user input from the user/patient indicative of identifying data, contact data, and/or the like, which may be provided to the monitoring system 40 for storage thereon.

### B. Patient Monitoring and Data Collection

In certain embodiments, the monitoring system 40 may be configured to generate at least a portion of health reports based at least in part on patient data received from a wearable client device 14. Accordingly, the wearable client device 14 of certain embodiments is configured to generate patient data based at least in part on onboard and/or external sensors, testers, and/or other data sources as discussed herein.

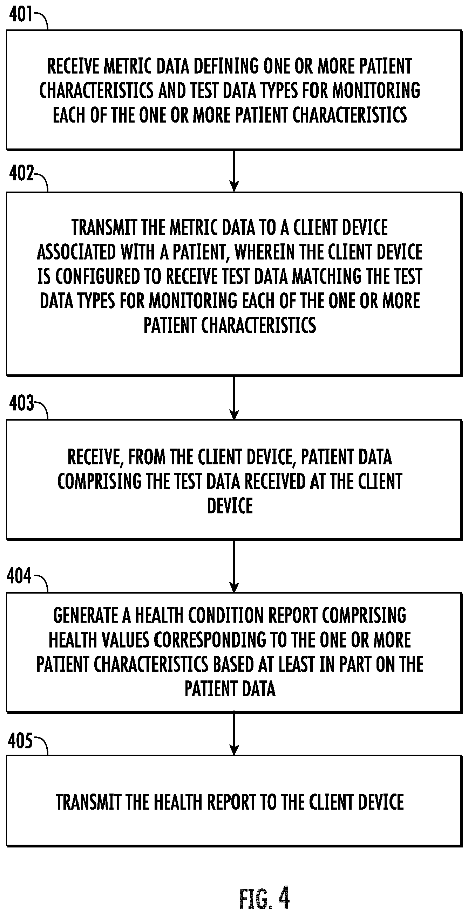

Moreover, the monitoring system 40 is configured to ensure that appropriate and/or necessary patient data is collected/generated at the wearable client device 14 to enable generation of the health report with the requested contents for the user/patient. With reference briefly to Fig. 4, which illustrates various steps performed by a monitoring system according to certain embodiments, the monitoring system 40 may receive and/or generate metric data, as indicated at Block 401 of Fig. 4, indicative of the patient characteristics and test data types (e.g., particular raw patient data types) required for generation of the health report data for the user/patient. In certain embodiments, receiving and/or generating the metric data may comprise receiving user input specifying the types of patient data necessary for generation of certain health report data. In various embodiments, the user input identifying necessary patient data (and/or providing metric data) may provide data to be utilized as a proxy for the patient data that may be required for generation of certain health report data. As just one example, user input may specify the types of tests necessary for retrieving patient data necessary for generation of particular health report data (e.g., providing a report of a patient's blood glucose level may require the patient to undergo blood sugar testing). The monitoring system 40 may then be configured to correlate the type of patient data generated by the identified testing to generate the metric data for the user/patient. In other embodiments, the user input may directly specify the metric data necessary for generation of the patient's requested health report data.

In other embodiments, the monitoring system 40 may be configured to automatically generate metric data based at least in part on data identifying health report contents requested. In certain embodiments, the monitoring system 40 may comprise an index stored within a memory storage area correlating metric data with particular heath report components. The index may be generated based at least in part on user input from healthcare professionals 15 (as discussed above, the user input specifying metric data necessary for generation of health report components). In other embodiments, the index may be at least partially generated automatically, for example, via a machine-learning model trained utilizing pairs of historical health reports and patient data corresponding to the historical health reports, such that the machine-learning model may identify patient data utilized to generate the particular historical health reports.

Accordingly, the metric data may be generated based at least in part on user input data requesting the particular type of data to be included within a health report. In other embodiments however, the report data to be included within the health report may also be automatically generated, for example, based at least in part on a state of the patient, a state of the patient's environment, a monitoring model, and/or the like. For example, the monitoring system 40 may be configured to utilize patient data received from a wearable client device 14 to determine an initial (or updated) state of the patient. For example, the state of the patient may be indicative of particular health conditions associated with the patient (such patient data may be manually input into the wearable client device 14 in certain embodiments, and/or such patient data may be automatically generated at the wearable client device 14). In other embodiments, the monitoring system 40 may be configured to perform an initial analysis of the received patient data to determine a state of the patient, and then the monitoring system 40 may be configured to generate metric data based at least in part on the determined state of the patient. For example, the monitoring system 40 may be configured to execute a monitoring model, which correlates a particular state of a patient with particular metric data. As yet another example, the monitoring system 40 may be configured to generate and/or determine data indicative of a state of the patient's environment, such as a determination of the location of the patient (e.g., based at least in part on patient identifying data), and to generate metric data based at least in part on the state of the patient's environment, for example, by executing a monitoring model correlating a state of the patient's environment with metric data.

As reflected in Block 402 of Fig. 4, the monitoring system 40 transmits metric data to the wearable client device 14 for local storage thereon. The metric data stored on the wearable client device 14 may be utilized by the wearable client device 14 to determine the type of patient data to be generated and/or collected. In certain embodiments, the wearable client device 14 may be configured to operate and/or cause operation of one or more onboard and/or external sensors, testers, and/or the like to generate patient data to satisfy requirements specified by the metric data. For example, upon determining that the metric data specifies that patient data indicative of a user/patient's pulse is necessary for generation of at least a portion of a health report, the wearable client device 14 may cause an onboard pulse monitoring sensor to collect patient data indicative of a patient's pulse. As a more specific example, if the metric data specifies that a daily average of a user/patient's pulse is necessary, the wearable client device 14 may be configured to cause an onboard pulse monitoring sensor to continuously and/or periodically monitor a patient's pulse over the course of a day, and the wearable client device 14 may then calculate a daily average pulse based at least in part on the data generated by the onboard pulse sensor.

In certain embodiments, particularly those embodiments in which the metric data specifies that a particular patient data type is required that cannot be collected automatically utilizing onboard sensors of the wearable client device 14 and/or utilizing external sensors that are in electronic communication with the wearable client device 14, the wearable client device 14 may be configured to generate one or more alerts to be displayed via an onboard display device to the user/patient indicating what additional patient data is necessary for generation of the requested reports. For example, the alerts may request that the user/patient manually provide the additional needed patient data, and/or the alerts may request that the user/patient electronically connect a sensor capable of generating the necessary patient data, such that the wearable client device 14 may receive and/or collect the necessary patient data to enable generation of the requested health report data.

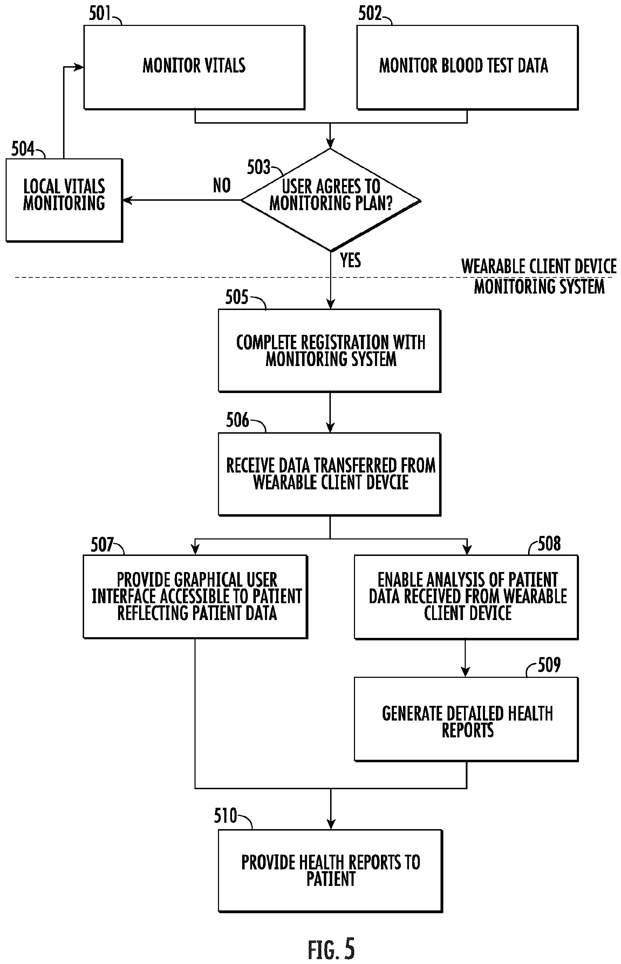

With reference now to Fig. 5, which illustrates operations of various components for generation of a health report according to various embodiments, the wearable client device 14 is configured to collect/generate/receive the patient data as specified within the metric data. As shown specifically in Fig. 5, for example, the wearable client device 14 according to one embodiment may monitor a user/patient's vitals (as reflected at Block 501), for example, via one or more onboard sensors. The wearable client device 14 may additionally monitor blood test data (as reflected at Block 502), or other patient data. In certain embodiments, at least a portion of the patient data, such as the blood test data utilized for monitoring by the wearable client device 14, may be generated by an external sensor or tester (e.g., which may be physically or wirelessly connected with the wearable client device 14).

As also reflected within Fig. 5, further processing of the patient data (and indeed identification of the type of patient data to be collected) may be informed by a determination of whether the user/patient has registered and/or has requested additional analysis to be performed at least in part via/through the monitoring system 40-as reflected at Block 503. The wearable client device 14 of certain embodiments may be configured for entirely local operation (e.g., with no data communication to a monitoring system 40 or any other computing device; with data communication only to defined computing devices, such as the user/patient's own client device(s) 10A-10N). Accordingly, if the user is not registered and/or has not requested generation of health reports utilizing the monitoring system 40, the wearable client device 14 may be configured to continue monitoring only certain specified data (e.g., as specified by metric data that may be locally generated and/or stored at the wearable client device 14), such as patient vital data that may be monitored via onboard sensors of the wearable client device 14.

As reflected in Fig. 5, upon a determination that the user has registered and/or has requested analysis performed at least in part utilizing the monitoring system 40, the wearable client device 14 is configured to transmit collected patient data to the monitoring system 40 for further analysis. In certain embodiments, the monitoring system 40 may be configured to correlate the patient data with stored patient identifying data, for example, utilizing metadata indicative of the source wearable client device 14 transmitted with the patient data. As other examples, the monitoring system 40 may be configured to correlate the patient data with stored patient identifying data utilizing other transmitted data, such as a portion of the patient data itself.

If not previously completed, the monitoring system 40 is configured to complete the registration process, as reflected in Block 505. As should be understood, registration may be completed as a part of an initial patient data transmission. However, it should be understood that in certain embodiments, registration may be completed via a separate registration process to be completed prior to the initial transmission of patient data to the monitoring system 40.

Ultimately, as reflected at Block 403 of Fig. 4 and Block 506 of Fig. 5, the patient data is received at the monitoring system 40 from the wearable client device 14 for further analysis. In certain embodiments, at least a portion of the analysis of the patient data may be performed automatically at the monitoring system 40. Moreover, as a portion of the analysis, the monitoring system 40 may be configured to generate a graphical user interface accessible to the user/patient reflecting the patient data as indicated at Block 507. The graphical user interface may comprise the raw patient data collected by the wearable client device 14 and/or may comprise data indicative of the sensors utilized to generate the raw patient data. In certain embodiments, the graphical user interface may enable the user/patient to view historical patient data, for example, by selecting a date/time for which the patient data is to be viewed/retrieved.

Performing analysis of the patient data may comprise steps for enabling analysis of the patient data (as reflected at Block 508). Enabling analysis of the patient data may comprise performing automated analysis at the monitoring system 40 of at least a portion of the patient data. As examples, a determination of whether a patient's pulse is dangerously high may be performed automatically and/or a determination of whether a patient's body temperature is dangerously high may be performed automatically based on rules/algorithms/thresholds stored by the monitoring system 40 and utilized for analyzing pulse data included within received patient data. In certain embodiments, processes for automatically analyzing patient data may comprise generating patient health report data to be utilized to generate at least a portion of a patient health report. Generation of the patient report data may comprise generating data indicative of the results of the automatically performed analysis to be included within the patient health report (as discussed below).

In certain embodiments, enabling analysis of the patient data may comprise providing at least a portion of the patient data to one or more client devices 10A-10N associated with healthcare professionals. In certain embodiments, providing at least a portion of the patient data to one or more client devices 10A-10N may comprise enabling access to at least a portion of a patient database by one or more client devices 10A-10N based at least in part on authorization credentials associated with the healthcare professionals operating the client devices 10A-10N. As discussed above, access to the patient data may be limited based at least in part on a user/patient's privacy preferences, such that only healthcare professionals (and their associated client devices 10A-10N) satisfying applicable privacy criteria are given access to the patient data. For example, patient data stored within a database associated with the monitoring system 40 may have associated metadata identifying privacy criteria applicable to the particular patient data. Authorization data associated with healthcare professionals may have corresponding professional data that may be utilized to determine whether the healthcare professional satisfies the privacy criteria associated with the patient data. As noted above, the privacy criteria may specify types of healthcare professionals that may access the patient data, names/identifiers of specific healthcare professionals that may access the data, locations of particular healthcare professionals that may access the data, specialties of particular healthcare professionals that may access the data, and/or the like. Thus, the authorization data associated with each healthcare professional may comprise identifying data of the healthcare professional (e.g., the name and/or identifier of the healthcare professional), a type of healthcare professional, the specialty/specialties associated with the healthcare professional, the location of the healthcare professional, and/or the like. Thus, only that patient data having privacy criteria satisfied by the healthcare professional's authorization data is made available for review by the healthcare professional.

Moreover, in certain embodiments, a patient's own patient data may have a single set of privacy criteria applicable to all of the patient's own patient data (e.g., thereby limiting healthcare providers to viewing none or all of the patient data based on the applicable privacy criteria). In other embodiments, privacy criteria may be set individually for particular portions of patient data. Thus, a first set of privacy criteria may apply to patient data that may be utilized to uniquely identify the patient, and a second set of privacy criteria may apply to anonymized patient data that cannot be utilized to uniquely identify the patient.

In various embodiments, enabling healthcare professionals to access the patient data may enable a more personal analysis of the patient data, even when the healthcare professional does not formally meet the patient and/or order the generation of the patient data. Thus, a healthcare professional may utilize his/her own medical judgment to determine whether any of the patient data is indicative of medical issues that should be flagged for the user/patient. In the event that the healthcare professional analyzes anonymized patient data, the healthcare professional's analysis may be matched with patient identifying data after submission to the monitoring system 40 (e.g., based at least in part on metadata associated with the anonymized patient data provided to the healthcare professional's client device 10A-10N, and that metadata matching at least a portion of the patient identifying data).

The data reflecting the results of the analysis of the patient data, whether performed automatically by the monitoring system 40 and/or manually by one or more healthcare professionals, may be presented to the monitoring system 40 for assembly of a health report for the user/patient.

### C. Generation of Health Reports

In certain embodiments, the monitoring system 40 may be configured to generate one or more detailed health reports for a user/patient, as reflected at Block 404 of Fig. 4 and Block 509 of Fig. 5. The detailed health reports may comprise the results of analysis of patient data as performed, for example, by the monitoring server 40 (e.g., automatically) and/or by one or more healthcare professionals.

In certain embodiments, the detailed health reports for a user may be embodied as a graphical user interface providing data in various forms, such as in graph-based forms (e.g., shown a bar graph, line graph, plot, and/or the like), alphanumeric text based forms (e.g., providing text indicative of a particular health value for a particular characteristic (e.g., a blood pressure value, a blood glucose level value, a pulse value, and/or the like); providing text describing risk factors of a user/patient (e.g., indicating that blood pressure is higher than a healthy range); providing text describing potential diseases/conditions for which additional testing is advisable (e.g., text indicating that further diabetes testing is advisable); and/or the like).

In various embodiments, the graphical user interface may be accessible to a user/patient via a computing device, such as wearable client device 14 and/or other client devices 10A-10N (e.g., utilizing user identifying data to access the patient's health report).

As discussed herein, the contents of a patient's health report may be selected based at least in part on user input identifying contents to be included within the patient's health report, based at least in part on patient data collected for the patient, based at least in part on user input from a healthcare professional identifying recommended contents of the health report, and/or the like. Accordingly, the monitoring system 40 generates the health report based at least in part on stored data identifying the contents to be included within the health report. The stored data may identify the types of content to be included within the health report, and accordingly the monitoring system 40 may be further configured to retrieve data necessary to populate each type of content to be included within the health report, for example, based at least in part on the results of the analysis performed on the patient data. Upon generation of the health report, the monitoring system 40 is configured to provide the health report to the user/patient, for example by transmitting the health report to a client device (e.g., a client device 10A-10N and/or the wearable client device 14) associated with the user/patient, as reflected in Block 405 of Fig. 4 and Block 510 of Fig. 5.

### V. Conclusion

Many modifications and other embodiments will come to mind to one skilled in the art to which this disclosure pertains having the benefit of the teachings presented in the foregoing descriptions and the associated drawings. Therefore, it is to be understood that the disclosure is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A computer-implemented method, the method comprising:
receiving metric data defining one or more patient characteristics and test data types for monitoring each of the one or more patient characteristics;
transmitting the metric data to a client device associated with a patient, wherein the client device is configured to receive test data matching the test data types for monitoring each of the one or more patient characteristics;
receiving, from the client device, patient data comprising the test data received at the client device;
generating a health report comprising health values corresponding to the one or more patient characteristics based at least in part on the patient data; and
transmitting the health report to the client device.

2. The method of claim 1, wherein the client device is a wearable client device.

3. The method of any one of claims 1-2, wherein the wearable client device is configured for generating at least a portion of the test data.

4. The method of any one of claims 1-3, further comprising:
updating the metric data based at least in part on a state of the patient, a state of an environment of the patient, and a monitoring model; and
transmitting the updated metric data to the client device.

5. The method of any one of claims 1-4, wherein the test data types comprise one or more of blood test results and vitals monitoring.

6. The method of any one of claims 1-5, further comprising:
generating a health report comprising presenting at least a portion of the patient data to a healthcare professional;
receiving input from the healthcare professional; and
determining health values based at least in part on the input.

7. The method of claim 6, further comprising:
identifying privacy criteria associated with the patient; and
determining authorization data associated with the healthcare professional and the privacy criteria indicate the healthcare professional is permitted access to the health report.

8. The method of claim 6, wherein the health report comprises an anonymized portion of the patient data.

9. The method of any one of claims 1-8, wherein the patient data is identified by a patient identifier identifying the patient at a monitoring system.

10. The method of claim 9, further comprising:
providing monitoring services to a plurality of users in a plurality of test data processing steps provided by the monitoring system, resulting in a plurality of health report views.

11. The method of any one of claims 1-10, further comprising:
receiving initial patient data associated with the patient;
determining, from the initial patient data, an initial health state associated with the patient; and
determining the metric data from the initial health state.

12. The method of any one of claims 1-11, further comprising:
receiving initial patient data associated with the patient;
determining, from the initial patient data, a patient environment associated with the patient; and
determining the metric data from the patient environment.

13. The method of any one of claims 1-12, further comprising:
receiving metadata indicative of the client device; and
correlating the patient data with stored patient identifying data representing the patient utilizing the client device.

14. An apparatus comprising at least one processor and at least one memory including computer program code, the at least one memory and the computer program code configured to, with the at least one processor, cause the apparatus to perform any one of the methods of claims 1-13.

15. A computer program product comprising at least one non-transitory computer-readable medium having computer-readable program instructions stored therein, the computer-readable program instructions comprising instructions, which when performed by an apparatus, are configured to cause the apparatus to at least perform any one of the methods of claims 1-13.
